# FASCICULE DE BREVET EUROPEEN

(11) **EP 0 609 210 B1**
(45) Date de publication et mention de la délivrance du brevet: **12.04.1995**
(21) Numéro de dépôt: 92903994.9
(22) Date de dépôt: 16.01.1992
(51) Int. Cl.: C07D 487/04, C07B 57/00, A61K 31/50

(54) **DERIVE DE LA 5H-PYRROLO[3,4-b]PYRAZINE OPTIQUEMENT ACTIF, SA PREPARATION ET LES COMPOSITIONS PHARMACEUTIQUES QUI LE CONTIENNENT**
OPTISCH AKTIVES 5H-PYRROLO[3,4-b]PYRAZINDERIVAT, SEINE HERSTELLUNG UND PHARMAZEUTISCHE ZUBEREITUNGEN DIE ES ENTHALTEN
OPTICALLY ACTIVE 5H-PYRROLO[3,4-b]PYRAZINE DERIVATIVE, ITS PREPARATION AND PHARMACEUTICAL COMPOSITIONS CONTAINING IT

(30) Priorité: 17.01.1991 FR 9100490
(43) Date de publication de la demande: 10.08.1994
(73) Titulaire: RHONE-POULENC RORER S.A., 92160 Antony (FR)
(72) Inventeur: COTREL, Claude, F-75012 Paris (FR); ROUSSEL, Gérard, F-91450 Soisy-sur-Seine (FR)
(74) Mandataire: Pilard, Jacques
(86) Numéro de dépôt international: FR9200031
(87) Numéro de publication internationale: WO9212980

(56) Documents cités:
- DE-A- 2 300 491
- US-A- 4 220 646
- "The Merck Index", 11eme Edition, 1989 , Merck & Co., Inc., Rathway, N.J., USA, résumé 10095

## Description

Dans le brevet français FR 72 00505 publié sous le numéro 2 166 314 a été décrite en particulier la (chloro-5 pyridyl-2)-6 (méthyl-4 pipérazinyl-1) carbonyloxy-5 oxo-7 dihydro-6,7 5H-pyrrolo [3,4-b] pyrazine, connue également sous le nom de zopiclone, qui est un produit hypnotique remarquable.

Du fait de la présence d'un atome de carbone asymétrique en position -5 du cycle 5H-pyrrolo[3,4-b] pyrazine, la zopiclone doit être considérée, sous forme racémique, comme étant constituée d'un mélange rigoureusement équimoléculaire des formes lévogyre et dextrogyre.

Il a maintenant été trouvé, et c'est ce qui fait l'objet de la présente invention, que l'isomère dervogyre de la zopiclone présente des propriétés qui ne sont pas évidentes au vu de celles de la zopiclone racémique.

La présente invention a donc pour objet l'isomère dextrogyre de la zopiclone, sa préparation et les compositions pharmaceutiques qui le contiennent. Dans un produit racémique, il est connu que souvent un des deux énantiomères est actif et que à cette activité peut être liée un accroissement de la toxicité, l'autre énantiomère étant à la fois nettement moins actif ou inactif et moins toxique. Pour de tels produits, le gain d'activité ne compense pas les inconvénients dus à une toxicité accrue.

Dans le cas de la zopiclone, il a été trouvé, de manière surprenante et inattendue, que l'isomére dextrogyre est non seulement environ 2 fois plus actif que le racémique tout en ayant une toxicité plus faible que celle du racémique et que l'isomère lévogyre est à la fois pratiquement inactif et plus toxique que le racémique. Par exemple, chez la souris, par voie orale, la zopiclone présente une toxicité

(DL₅₀) voisine de 850 mg/kg alors que l'isomère dextrogyre a une toxicité voisine de 1,5 g/kg et que l'isomère lévogyre présente une DL₅₀ comprise entre 300 et 900 mg/kg.

Chez l'animal, l'isomère dextrogyre de la zopiclone montre des propriétés hypnotique, sédative, anxiolytique, myorelaxante et anticonvulsivante.

Du point de vue de la puissance d'action dans les principaux tests mettant en évidence l'activité tranquillisante et hypnotique de la zopiclone tels que le test d'affinité vis-à-vis des sites récepteurs centraux des benzodiazépines selon la technique de J.C. Blanchard et L. Julou, J. of Neurochemistry, 40, 601 (1983) inspirée des travaux de Squires et Braestrup, Nature, 266, 732-734 (1977), le test d'activité antagoniste vis-à-vis des convulsions au pentétrazol selon la technique de Everett et Richards, J. Pharmacol., 81, 402 (1944), ou dans le test du réflexe de retournement chez la souris selon la technique de Zbinden et Randall, Advances in Pharmacology 5, 213-291 (1967), l'isomère dextrogyre est environ 2 fois plus actif alors que l'isomère lévogyre est pratiquement inactif.

Selon l'invention, l'isomère dextrogyre de la zopiclone peut être préparé à partir du racémique correspondant selon les méthodes habituelles telles que la chromatographie sur phase chirale, le dédoublement d'un sel optiquement actif ou la catalyse enzymatique stéréosélective au moyen d'un microorganisme convenable ou par synthèse asymétrique.

Plus particulièrement, l'isomère dextrogyre de la zopiclone peut être obtenu par dédoublement de la zopiclone au moyen d'un acide optiquement actif en opérant dans un solvant organique convenable.

Comme acide optiquement actif qui convient particulièrement bien peut être cité l'acide D(+)-O,O'-dibenzoyltartrique.

Généralement, on opère dans un solvant organique choisi parmi les hydrocarbures aliphatiques halogénés tels que le dichlorométhane et les nitriles tels que l'acétonitrile pris seuls ou en mélange.

En opérant de cette manière, le sel de l'isomère dextrogyre précipite et l'isomère lévogyre est extrait des eaux-mères de cristallisation.

L'isomère dextrogyre de la zopiclone est déplacé de son sel au moyen d'une base telle que la soude.

L'isomère dextrogyre de la zopiclone est utile chez l'homme pour le traitement des états dus à un disfonctionnement du système nerveux central.

L'isomère dextrogyre de la zopiclone est, par exemple, utile comme hypnosédatif, tranquillisant, myrorelaxant et anticonvulsivant.

Cependant, l'isomère dextrogyre de la zopiclone est plus particulièrement utile chez l'homme comme hypnotique.

L'isomère dextrogyre de la zopiclone agissant sur les divers paramètres du sommeil, il augmente la durée et améliore la qualité du sommeil et il diminue le nombre d'éveils nocturnes et de réveils précoces.

La présente invention concerne les compositions pharmaceutiques contenant l'isomère dextrogyre de la zopiclone ou un de ses sels pharmaceutiquement acceptables à l'état pur ou en présence d'un diluant ou d'un enrobage. Ces compositions peuvent être employées par voie orale, rectale ou parentérale.

Comme sels pharmaceutiquement acceptables peuvent être cités des sels d'acides minéraux (tels que chlorhydrates, sulfates, nitrates, phosphates) ou organiques (tels que les acétates, propionates, succinates, benzoates, fumarates, tartrates, théophyllineacétates, salicylates, phénolphtalinates, méthylène-bis-β-oxynaphtoates) ou des dérivés de substitution de ces acides.

Comme compositions solides pour administration orale peuvent être utilisés des comprimés, des pilules, des poudres ou des granulés. Dans ces compositions le produit actif selon l'invention est mélangé à un ou plusieurs diluants inertes, tels que saccharose, lactose ou amidon. Ces compositions peuvent également comprendre des substances autres que les diluants, par exemple un lubrifiant tel que le stéarate de magnésium.

Comme compositions liquides pour administration orale on peut utiliser des émulsions pharmaceutiquement acceptables, des solutions, des suspensions, des sirops et élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale peuvent être des solutions stériles aqueuses ou non aqueuses, des suspensions ou des émulsions. Comme solvant ou véhicule, on peut employer le propylèneglycol, le polyéthylèneglycol, les huiles végétales, en particulier l'huile d'olive, et les esters organiques injectables, par exemple l'oléate d'éthyle. Ces compositions peuvent contenir des adjuvants en particulier des agents mouillants, émulsifiants et dispersants. La stérilisation peut se faire de plusieurs façons, par exemple à l'aide d'un filtre bactériologique, en incorporant à la composition des agents stérilisants, par irradiation ou par chauffage. Elles peuvent être préparées sous forme de compositions stériles qui peuvent être dissoutes au moment de l'emploi dans de l'eau stérile ou tout autre milieu stérile injectable.

Les compositions pour administration rectale sont des suppositoires qui peuvent contenir, outre le produit actif, des excipients tels que le beurre de cacao.

En thérapeutique humaine les doses dépendent de l'effet recherché et de la durée du traitement ; elles sont généralement comprises entre 2,5 et 15 mg par jour par voie orale pour un adulte.

Les exemples suivants, donnés à titre non limitatif, illustrent la présente invention.

### EXEMPLE 1

A une solution de 22,56 g d'acide D(+)-O,O'-dibenzoyltartrique sous forme de monohydrate (0,06 mole) dans 300 cm3 de dichlorométhane, on ajoute une solution de 23,28 g de zopiclone (0,06 mole) dans 300 cm3 de dichlorométhane. Le mélange réactionnel est concentré à sec sous pression réduite. Le sel brut obtenu est recristallisé dans 2000 cm3 d'acétonitrile pour donner, avec un rendement de 46 %, 21,3 g de produit cristallisé fondant à 160-165°C (avec décomposition) et dont le pouvoir rotatoire est [α]²⁰_{D} = 83° (c= 0,5 ; acétone).

Le produit obtenu est dissous dans 180 cm3 de dichlorométhane au reflux. On ajoute 200 cm3 d'acétonitrile et laisse reposer pendant 1 heure à une température de 5°C. Le produit cristallisé obtenu est à nouveau recristallisé dans les mêmes conditions. On obtient ainsi, avec un rendement de 36 %, 16,5 g de sel cristallisé fondant à 160-165°C (avec décomposition) et dont le pouvoir rotatoire est [α]²⁰_{D} = 102° (c = 0,5 ; acétone).

Le sel ainsi obtenu est dissous dans 125 cm3 d'eau en présence de 125 cm3 de dichlorométhane. Le mélange est alcalinisé à pH 11 par addition lente d'une solution aqueuse de soude 2N. Après décantation, la phase aqueuse est extraite deux fois avec du dichlorométhane. Les phases organiques réunies sont lavées à l'eau puis séchées sur sulfate de magnésium. Après filtration, évaporation du solvant et recristallisation du produit obtenu dans 80 cm3 d'acétonitrile, on obtient, avec un rendement de 23 %, 5,4 g d'isomère dextrogyre de la zopiclone fondant à 206,5°C et dont le pouvoir rotatoire est [α]²⁰_{D} = 135° ± 3° (c = 1,0 ; acétone).

Les eaux-mères de cristallisation du sel de la zopiclone avec l'acide D(+)-O,O'-dibenzoyltartrique sont concentrées à sec sous pression réduite pour donner 22,05g de sel dont le pouvoir rotatoire est [α]²⁰_{D} = -21° (c = 0,2 ; acétone).

Le sel ainsi obtenu est dissous dans 125 cm3 d'eau en présence de 125 cm3 de dichlorométhane. Le mélange est alcalinisé à pH 11 par addition lente d'une solution aqueuse de soude 2N. Après décantation, la phase aqueuse est extraite deux fois avec du dichlorométhane. Les phases organiques réunies sont lavées à l'eau puis séchées sur sulfate de magnésium. Après filtration et évaporation du solvant, le solide cristallisé obtenu (8,45 g) est recristallisé successivement dans 100, 50 et 45 cm3 d'acétonitrile. On obtient ainsi, avec un rendement de 13,9 %, 3,13 g d'isomère lévogyre de la zopiclone fondant à 206,9°C et dont le pouvoir rotatoire est [α]²⁰_{D} = -133° ± 3° (c = 1,0 ; acétone).

### EXEMPLE 2

On prépare selon la technique habituelle des comprimés dosés à 3 mg de produit actif ayant la composition suivante :

| | |
|---|---|
| - isomère dextrogyre de la zopiclone | 0,003 g |
| - amidon | 0,100 g |
| - silice précipitée | 0,035 g |
| - stéarate de magnésium | 0,005 g |

## Revendications

1. L'isomère dextrogyre de la (chloro-5 pyridyl-2)-6 (méthyl-4 pipérazinyl-1) carbonyloxy-5 oxo-7 dihydro-6,7 5H-pyrrolo [3,4-b] pyrazine ainsi que ses sels pharmaceutiquement acceptables.

2. Procédé de préparation du produit selon la revendication 1 caractérisé en ce que l'on dédouble la (chloro-5 pyridyl-2)-6 (méthyl-4 pipérazinyl-1) carbonyloxy-5 oxo-7 dihydro-6,7 5H-pyrrolo[3,4-b]pyrazine racémique au moyen d'acide D(+)-O,O'-dibenzoyltartrique en opérant dans un solvant organique, isole le sel de l'isomère dextrogyre et déplace de son sel puis isole l'isomère dextrogyre de la (chloro-5 pyridyl-2)-6 (méthyl-4 pipérazinyl-1) carbonyloxy-5 oxo-7 dihydro-6,7 5H-pyrrolo[3,4-b]pyrazine que l'on transforme éventuellement en sel pharmaceutiquement acceptable.

3. Composition pharmaceutique caractérisée en ce qu'elle contient le produit selon la revendication 1 en association avec un ou plusieurs diluants ou adjuvants pharmaceutiquement acceptables.

## Claims

1. Dextrorotatory isomer of 6-(5-chloro-2-pyridyl)-5-[(4-methyl-1-piperazinyl)carbonyl-oxy]-7-oxo-6,7-dihydro-5H-pyrrolo[3,4-b]pyrazine, as well as its pharmaceutically acceptable salts.

2. Process for preparing the product according to claim 1, characterized in that racemic 6-(5-chloro-2-pyridyl)-5-[(4-methyl-1-piperazinyl)carbonyloxy]-7-oxo-6,7-dihydro-5H-pyrrolo[3,4-b]pyrazine is resolved by means of D(+)-O,O'-dibenzoyltartaric acid, working in an organic solvent, the salt of the dextrorotatory isomer is isolated and the dextrorotatory isomer of 6-(5-chloro-2-pyridyl)-5-[(4-methyl-1-piperazinyl)carbonyloxy]-7-oxo-6,7-dihydro-5H-pyrrolo[3,4-b]-pyrazine is displaced from its salt, then isolated and optionally converted to a pharmaceutically acceptable salt.

3. Pharmaceutical composition, characterized in that it contains the product according to claim 1 in combination with one or more pharmaceutically acceptable diluents or adjuvants.

## Patentansprüche

1. Rechtsdrehendes Isomer von 6-(5-Chlor-2-pyridyl)-(4-methyl-1-piperazinyl)-5-carbonyloxy-7-oxo-6,7-dihydro-5H-pyrrolo[3,4-b]pyrazin sowie seine pharmazeutisch akzeptablen Salze.

2. Verfahren zur Herstellung des Produktes nach Anspruch 1, dadurch gekennzeichnet, daß man das racemische 6-(5-Chlor-2-pyridyl)-(4-methyl-1-piperazinyl)-5-carbonyloxy-7-oxo-6,7-dihydro-5H-pyrrolo[3,4-b]pyrazin mit Hilfe von D(+)-O,O-Dibenzoylweinsäure spaltet, wobei man in einem organischen Lösungsmittel arbeitet, das Salz des rechtsdrehenden Isomers isoliert und dieses aus seinem Salz verdrängt, und anschließend das rechtsdrehende Isomer von 6-(5-Chlor-2-pyridyl)-(4-methyl-1-piperazinyl)-5-carbonyloxy-7-oxo-6,7-dihydro-5H-pyrrolo[3,4-b]pyrazin isoliert, das man gegebenenfalls in ein pharmazeutisch akzeptables Salz umwandelt.

3. Pharmazeutische Zusammensetzung, dadurch gekennzeichnet, daß sie das Produkt nach Anspruch 1 in Assoziation mit einem oder mehreren pharmazeutisch akzeptablen Verdünnungsmitteln oder Zusatzstoffen enthält.
